(19) Europäisches Patentamt
European Patent Office
Office européen des brevets

(11) Publication number : 0 606 747 A1

(12) # EUROPEAN PATENT APPLICATION

(21) Application number : 93310258.4

(22) Date of filing : 17.12.93

(51) Int. Cl.$^5$ : **A61K 31/445,** A61K 31/395, A61K 31/365

(30) Priority : 21.12.92 US 994579

(43) Date of publication of application : 20.07.94 Bulletin 94/29

(84) Designated Contracting States : AT BE CH DE DK ES FR GB GR IE IT LI LU NL PT SE

(71) Applicant : ELI LILLY AND COMPANY
Lilly Corporate Center
Indianapolis Indiana 46285 (US)

(72) Inventor : Ose, Earl Eugene
805 Oak Court
Greenfield, Indiana 46140 (US)

(74) Representative : Hudson, Christopher Mark et al
Erl Wood Manor
Windlesham Surrey GU20 6PH (GB)

(54) **Tilmicosin and derivatives for treating gram-positive diseases of aquatic species.**

(57) The invention is directed to the use of tilmicosin and related compounds for the treatment of gram-positive bacterial pathogens in aquatic species.

EP 0 606 747 A1

BRIEF SUMMARY OF THE INVENTION

Aquaculture is now producing a substantial portion of the world's protein. However, with its greater concentration of individual animals than in natural locations, aquaculture presents an increased need for therapeutics.

The salmonid fish species -- notably salmon and trout -- are susceptible to a disease known as "bacterial kidney disease." It is caused by a gram-positive organism identified as *Renibacterium salmoninarum.* This disease is one of the two most devastating diseases in the farming of salmonids. The perciform fishes are also subject to attack by gram-positive bacterial pathogens. As one example, yellowtail fish, a species which is farmed in Japan, suffers devastating losses from gram-positive bacteria of the *Streptococcus* genus. Also, the crustaceans which are produced in aquaculture are subject to gram-positive pathogens.

Although a limited number of therapeutics are used in aquaculture, none is entirely satisfactory. Transmission of disease from one animal to another is facilitated readily by the fluid medium, more so than for land species, and losses can be very high. The still-young aquaculture industry therefore needs additional tools to deal with infections.

The present invention provides a new therapeutic tool, the use of the compounds defined by U.S. patent 4,820,695, for the control of gram-positive bacterial species in salmonid and perciform fishes as well as in crustaceans.

DETAILED DESCRIPTION OF THE INVENTION

U.S. patent 4,820,695, which is incorporated herein by reference, describes certain macrolide antibiotics. The description of the compounds is duplicated for the benefit of the reader, as follows:

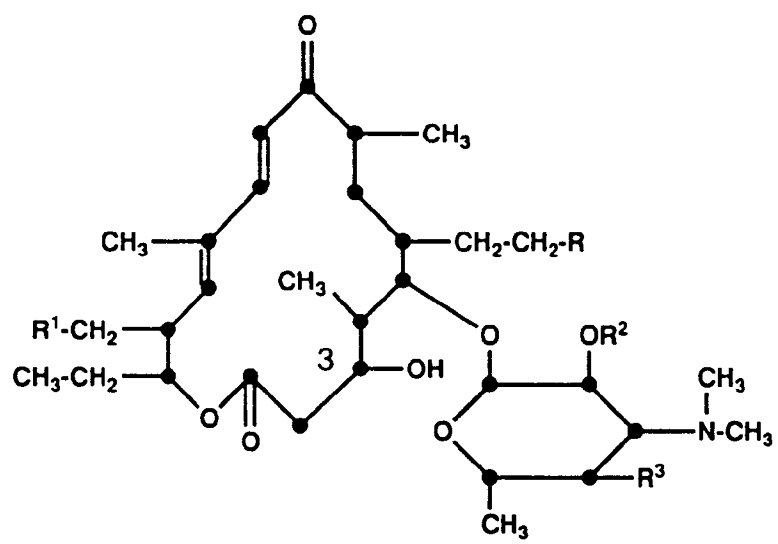

wherein

R is a saturated or unsaturated secondary amino group of the formula

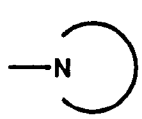

in which the nitrogen atom is part of an otherwise carbocyclic ring system selected from a monocyclic ring containing from 5 to 16 ring atoms or a bicyclic or tricyclic ring system containing from 8 to 20 ring atoms or such a group wherein one or more of the carbon atoms is substituted by $C_1$-$C_4$ alkyl, $C_2$-$C_4$ alkenyl, $C_2$-$C_4$ alkynyl, $C_1$-$C_4$ alkoxy, $C_1$-$C_4$ alkoxycarbonyl, hydroxyl, $C_1$-$C_4$ alkanoyloxy, halo, halo-$C_1$-$C_4$ alkyl, $N(C_1$-$C_4$ alkyl$)_2$, $-N(CH_2)_m$,

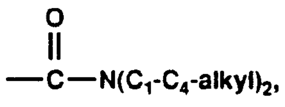, 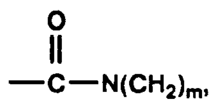,

cyano, ethylenedioxy, benzyl, phenyl, or phenyl substituted by from 1 to 3 substituents selected from nitro, halo, $C_1$-$C_4$-alkyl, $C_1$-$C_4$ alkoxy, hydroxy, amino, or mono- or di-($C_1$-$C_4$ alkyl)amino;

m is an integer from 4 through 7;

$R^1$ is

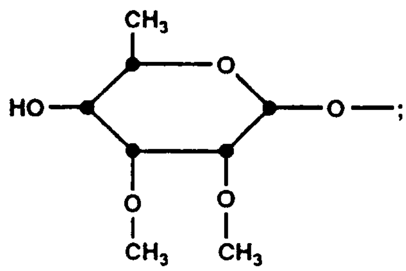;

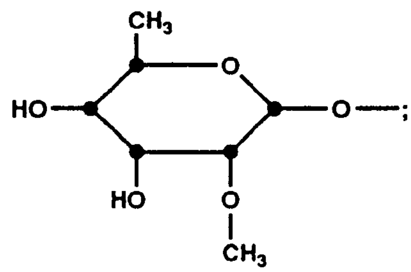;

or

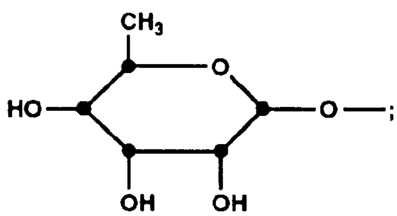;

$R^2$ is hydrogen; $C_1$-$C_5$-alkanoyl or $C_1$-$C_5$-alkanoyl having from one to three halo substituents; benzoyl, phenylacetyl or phenylpropionyl or benzoyl, phenylacetyl or phenylpropionyl having from one to five halo or methyl or from one to two methoxyl, nitro or hydroxyl substituents;

$R^3$ is hydroxy; $C_1$-$C_5$ alkanoyloxy; $C_1$-$C_5$-alkanoyloxy having from one to three halo substituents; benzoyloxy, phenylacetoxy or phenoxyacetoxy or benzoyloxy, phenylacetoxy or phenoxyacetoxy having from one to five halo or methyl or from one to two methoxyl, nitro or hydroxyl substituents; or

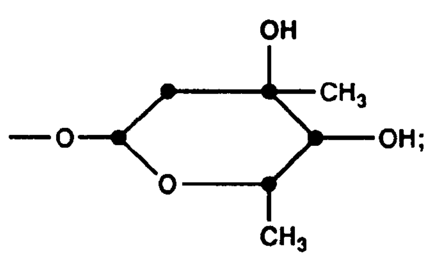;

and the acid addition salts thereof.

One compound from within 4,820,695 is known by the generic name tilmicosin, assigned by USAN, the United States Adopted Names organization. It has the following structural formula:

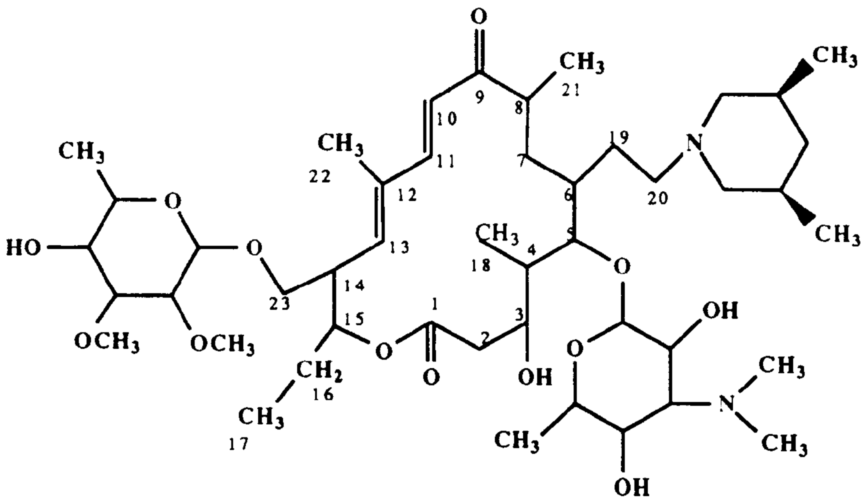

It has now been discovered that the compounds of 4,820,695, and tilmicosin in particular, can be used to control gram-positive bacterial diseases in aquaculture of several classes of aquatic organisms, notably the salmonids, the perciforms, and crustaceans. A compound of 4,820,695 may be used as such or in the form of a physiologically acceptable salt.

The salmonids are members of the Family *Salmonidae*, including salmon, trout, and char. Specific salmon include:

Atlantic salmon *(Salmo salar)*
Amago salmon *(Oncorhynchus rhodurus)*
Chum salmon *(Oncorhynchus keta)*
Masu salmon *(Oncorhynchus masou)*
Coho salmon *(Oncorhynchus kisutch)*
Pink salmon *(Oncorhynchus gorbuscha)*
Sockeye salmon *(Oncorhynchus nerka)*
Chinook salmon *(Oncorhynchus tshawytscha)*

Among the trout are

Brook trout *(Salvelinus fontinalis)*
Brown trout *(Salmo trutta)*
Rainbow trout *(Oncorhynchus mykiss)*
Lake trout *(Salvelinus namaycush)*

The most important char is

Arctic char *(Salvelinus alpinus)*

The perciform fishes are members of the Order *Perciformes,* including the Families *Pomotomidae, Centrarchidae, Percidae, Serranidae,* and *Cichlidae.* Specific perciform fishes grown in aquaculture include the following:

yellowtail
the various bass species
the various perches
walleye pike
sunfish
tilapia
mullet
bluefish

Finally, the crustaceans grown in aquaculture also suffer from gram-positive bacterial pathogens. Shrimp is the most prominent aquaculture-produced crustacean, but crayfish and crabs are also produced.

Gram-positive bacterial species which are pathogenic on aquatic species are numerous, even as for land species; but there are two which present the most severe challenge to the aquaculture industry: *Renibacterium salmoninarum* (bacterial kidney disease) in salmonids, and *Streptococcus* sp. in perciforms. Of lesser significance, but nonetheless troublesome, are *Beneckia* sp. in crabs and coryneform bacteria in septicemic bacterial disease syndrome in shrimp.

In the practice of the present invention, a compound of 4,820,695 is administered to the aquatic organism

in an amount effective to treat or prevent disease due to a gram-positive bacterial organism. The administration can be by any of the usual techniques of aquaculture. One common technique is that a therapeutic is administered orally by inclusion in a standard aquatic food. This food is distributed onto the surface of the body of water in which the aquatic species is being reared. Alternately, a therapeutic can be administered by immersing the aquatic species in a relatively concentrated solution of the therapeutic, for a short period of time. Also, administration can be made by injection. This route of administration is sometimes employed in controlling *Renibacterium salmoninarum,* which is transmitted from one generation to the next, through the eggs. Treatment of eggs is another available technique; the eggs are immersed in a bath which includes the therapeutic.

The amount of a compound of 4,820,695 to be employed in the present invention is not critical and will vary with the identity of the aquatic species, the identify of the bacterial species, the route of administration, and other factors known to those in the field of aquaculture. When the compound is administered via a feed, an effective amount will vary with the age and rate of consumption; however, concentrations in the diet of from 100 to 1000 ppm will generally provide an effective amount of the compound. In the instance of administration by injection, an effective amount of the compound is generally in the range of from 1 to 20 mg of compound per kilogram of body weight.

For use according to the present invention, a compound of 4,820,695 is typically formulated with one or more physiologically acceptable carriers, such as normal feed components, carriers for injection, and the like. The composition of aquatic feeds has been the subject of considerable study. When the present invention is administered via a feed, standard aquatic feeds can be employed. Such feeds are typically nutritionally balanced complete feeds appropriate for the species. See references such as Chapter 22 (perciforms) and Chapter 34 (salmonids) in Fish Medicine (Harcourt Brace Jovanovich, Inc., copyright 1993). The composition of crustacean diets is very similar to that of fish feeds. For purposes of the present invention, any of these conventional feeds can be modified to incorporate a compound of 4,820,695.

The present invention is illustrated by the following examples.

**Test 1: In vitro evaluation of tilmicosin against *Renibacterium salmoninarum.***

A dozen strains of *Renibactenum salmoninarum* were obtained and cultured in standard procedures. Eight grew successfully and were employed in the evaluation of tilmicosin.

Tilmicosin was evaluated at various concentrations and the minimum inhibitory concentration (MIC) determined for each of the strains. For three of the strains, the MIC was 1 μg/ml; for the remaining five strains, the MIC was 2 μg/ml. Even at lower concentrations down to 0.25 μg/ml, there was a dramatic reduction in growth.

**Test 2: Palatability and growth studies in Atlantic salmon.**

A trial was conducted to determine the palatability and effect on growth of Atlantic salmon *(Salmo salar)*, St. John River strain, of diets containing various concentrations of tilmicosin. Pre-smolt salmon, each weighing about 50 grams, from a stock certified to be free of bacterial kidney disease, were used. The fish were acclimatized for four weeks. During this period, the fish were in tanks maintained with fresh single-pass flow-through well water, 10±2°C., and a photoperiod of 12/12. Water flow rate was adequate to provide a dissolved oxygen concentration of ≧ 5 mg/liter post feeding and under conditions of normal activity provided a dissolved oxygen concentration of ≧ 8 mg/liter. All fish were fed a standard diet. During the last week of the acclimatization and thereafter during treatment, the fish were allocated to separate tanks, 25 fish per tank, and the water temperature was increased from 10±1°C. to 12±1°C.; the water flow rate was 1-4 liters/minute (average, 2.5 liters/minute). The same photoperiod was maintained.

Following acclimatization, treatment was begun. There were two tanks per treatment (four tanks for the control), and the treatments were as follows:

| tilmicosin concentration in feed |
|---|
| - (control) |
| 200 mg/kg |
| 600 mg/kg |
| 1000 mg/kg |

All fish were fed 3× on weekdays and 1× or 2× on weekend days. With the exception of two of the control tanks, feeding was to satiation of body weight. The remaining two control tanks were used for a "paired group" with the fish receiving 600 mg/kg tilmicosin; these control fish received an amount of feed equivalent to the amount of feed consumed by the treated fish the preceding day. The trial lasted four weeks, and the following parameters were determined:

feed intake
feed intake as % of body weight
weight gain
weight gain as % of body weight
feed conversion

At the end of the four weeks, the fish were sacrificed and tissue from liver, muscle, and kidney analyzed for tilmicosin by an HPLC assay. For this assay, tilmicosin was extracted from the tissue by homogenizing it with methanol and centrifuging it to separate the precipitate from the methanol extract. The extract was diluted with salt solution and partitioned with $CCl_4$. The aqueous phase was then basified and the tilmicosin extracted into $CHCl_3$:hexane. The extract was evaporated to dryness and reconstituted for HPLC assay. Quantitative determination of tilmicosin was accomplished by reversed-phase HPLC with a phenylsilyl stationary phase and a U.V. absorption detector at 280 nm.

In addition, all fish were observed for their feeding response and for any signs of altered behavior or toxicity. Post-mortem examination was conducted on all of the fish at the end of the study.

The formulation of the feeds, and the results of the trial were as set forth below in Tables 1-5. Throughout the tables, mean values with the same alphabetic superscript were not statistically different at the 0.05 level.

Table 1. Composition of Basal Mix

| Basal Mix | % |
|---|---|
| Herring meal | 38.0 |
| Blood meal | 5.2 |
| Soybean meal | 8.0 |
| Brewer's dried yeast | 4.0 |
| Corn gluten meal | 5.0 |
| Poultry by-product | 3.0 |
| Dried whey | 6.0 |
| Vitamin premix | 2.0 |
| Mineral premix | 4.0 |
| Choline chloride | 0.2 |
| DL-methionine | 0.2 |
| | 75.6 |

Table 2. Composition of Feeds

| | Composition of Trial Feeds | | | | |
|---|---|---|---|---|---|
| | grams of component | | | | |
| | 0T** | | 200T** | 600T** | 1000T** |
| Basal Mix | 6048 | (75.6%) | 2268 | 2268 | 2268 |
| Tilmicosin premix* | 0 | (0.0%) | 75 | 225 | 375 |
| Wheat middlings | 1152 | (14.4%) | 357 | 207 | 57 |
| Herring oil | 800 | (10.0%) | 300 | 300 | 300 |
| | 8000 | (100.0%) | 3000 | 3000 | 3000 |

** T = mg of tilmicosin/kg of finished feed

* The tilmicosin premix was prepared by mixing 11.23 g of tilmicosin base (8.0 g of tilmicosin activity) with 988.77 g of wheat middlings.

Table 3. Feed Intake, Weight Gain, and Feed Conversion

A. Feed intake and feed intake as a percentage of body weight

| Mg. of Tilmicosin per Kg. of Diet | feed intake (grams) | | | | feed intake as %BW* | | | |
|---|---|---|---|---|---|---|---|---|
| | Rep1 | Rep2 | Mean | S.D. | Rep1 | Rep2 | Mean | S.D. |
| 0 | 370.2 | 376.6 | 373.4a | 3.2 | 1.41 | 1.45 | 1.43b | 0.02 |
| 200 | 375.5 | 391.8** | 383.7a | 8.2 | 1.39 | 1.45 | 1.42b | 0.03 |
| 600 | 363.7 | 372.5 | 368.1a | 4.4 | 1.45 | 1.43 | 1.44b | 0.01 |
| 1000 | 427.0 | 326.4 | 376.7a | 50.3 | 1.50 | 1.29 | 1.39b | 0.11 |

B. Weight gain and weight gain as a percentage of body weight

| Mg. of Tilmicosin per Kg. of Diet | weight gain (grams) | | | | weight gain (as %BW) | | | |
|---|---|---|---|---|---|---|---|---|
| | Rep1 | Rep2 | Mean | S.D. | Rep1 | Rep2 | Mean | S.D. |
| 0 | 337.1 | 338.2 | 337.7a | 0.6 | 35.7 | 35.5 | 35.6b | 0.1 |
| 200 | 352.4 | 370.2** | 361.3a | 8.9 | 36.4 | 38.1 | 37.3b | 0.9 |
| 600 | 334.1 | 360.4 | 347.3a | 13.2 | 37.0 | 37.4 | 37.2b | 0.2 |
| 1000 | 429.5 | 318.5 | 374.0a | 55.5 | 42.1 | 34.2 | 38.2b | 4.0 |

C. Feed conversion

| Mg. of Tilmicosin per Kg. of Diet | feed conversion | | | |
|---|---|---|---|---|
| | Rep1 | Rep2 | Mean | S.D. |
| 0 | 1.10 | 1.14 | 1.12a | 0.02 |
| 200 | 1.07 | 1.07 | 1.07a | 0.00 |
| 600 | 1.09 | 1.03 | 1.06a | 0.03 |
| 1000 | 0.99 | 1.05 | 1.02a | 0.03 |

* Feed consumption averaged 1.43% of body weight per day, corresponding to a tilmicosin dose of 2.9, 8.6, and 14.3 mg/kg of body weight per day.

** This tank contained 26 fish. Relevant data have been corrected to 25 animals.

Table 4. HPLC Assay of Fish Tissues

| Tilmicosin Concentration in Diet, ppm | Tilmicosin concentration, ppm in: | | |
|---|---|---|---|
| | liver | muscle | kidney |
| 0 | BLOQ* | BLOQ | BLOQ |
| 200 | 0.9 | 0.2 | 2.7 |
| 600 | 2.5 | 0.4 | 6.7 |
| 1000 | 4.1 | 0.9 | 14.5 |

* BLOQ = Below level of quantitation.

Table 5. Feed Intake, Weight Gain, and Feed Conversion, Paired Study

A. Feed intake and feed intake as a percentage of body weight*

| Mg. of Tilmicosin | feed intake (grams) | | | | feed intake as %BW | | | |
|---|---|---|---|---|---|---|---|---|
| per Kg. of Diet | Rep1 | Rep2 | Mean | S.D. | Rep1 | Rep2 | Mean | S.D. |
| 600 | 363.7 | 372.5 | $368.1^a$ | 4.4 | 1.45 | 1.43 | $1.44^b$ | 0.01 |
| 0 | 359.1 | 334.0 | $346.6^a$ | 12.6 | 1.33 | 1.26 | $1.29^b$ | 0.03 |

B. Weight gain and weight gain as a percentage of body weight

| Mg. of Tilmicosin | weight gain (grams) | | | | weight gain (as %BW) | | | |
|---|---|---|---|---|---|---|---|---|
| per Kg. of Diet | Rep1 | Rep2 | Mean | S.D. | Rep1 | Rep2 | Mean | S.D. |
| 600 | 334.1 | 360.4 | $347.3^a$ | 13.2 | 37.0 | 37.4 | $37.2^b$ | 0.2 |
| 0 | 378.7 | 309.1 | $343.9^a$ | 34.8 | 38.5 | 32.4 | $35.5^b$ | 3.1 |

C. Feed conversion

| Mg. of Tilmicosin | feed conversion | | | |
|---|---|---|---|---|
| per Kg. of Diet | Rep1 | Rep2 | Mean | S.D. |
| 600 | 1.09 | 1.03 | $1.06^a$ | 0.03 |
| 0 | 0.95 | 1.08 | $1.01^a$ | 0.07 |

* Feed intake of the control fish was slightly lower than feed intake of the treated fish, because on a few days the control fish did not finish the offered amount of feed.

Tables 1-5 establish that diets containing tilmicosin at 200, 600, and 1000 mg/kg were palatable to Atlantic salmon and were consumed at the same rate as the control feed. Treated fish grew as efficiently as the control fish. Post-mortem examination of all of the fish at the end of the study revealed no gross abnormalities.

**Test 3: Palatability and Growth Studies in Atlantic Salmon, Comparing Tilmicosin Base and Phosphate Salt**

A trial was conducted to determine the palatability to Atlantic salmon *(Salmo salar),* St. John River strain, of diets containing either tilmicosin (free base) or tilmicosin phosphate. Pre-smolt salmon, each weighing about 50 grams, from a certified disease free stock, were used. The salmon, 30 per tank and 9 tanks, were acclimatized for about two weeks. During this period and throughout the trial, the tanks were maintained with fresh single-pass flow-through well water, 2 liters per minute, 12 ± 1°C., and the photoperiod was 12/12. During the acclimatization, all fish were fed a standard feed. Thereafter the salmon in three tanks continued to receive the standard diet, to serve as a control. The salmon in three other tanks received a modified diet containing 800 mg of tilmicosin (free base) per kilogram of diet; the salmon in the last three tanks received a modified diet containing tilmicosin phosphate in an amount equivalent to 800 mg of tilmicosin (free base) per kilogram of diet. All fish were fed to satiation, defined as the point at which there is a noticeable decline in the feeding response. Feed was supplied 3× on weekdays and 1× on weekend days. The trial was continued for a four-week period, during which these parameters were evaluated:

feed intake
feed intake as % of body weight
weight gain
weight gain as % of body weight
feed conversion

All surviving fish were euthanized. The results are set forth in the following table; mean values with the same alphabetic superscript were not statistically different at the 0.05 level.

Table 6. Composition of Feeds

| Diet composition | % |
|---|---|
| Herring meal | 38.0 |
| Blood meal | 5.2 |
| Soybean meal | 8.0 |
| Brewer's dried yeast | 4.0 |
| Corn gluten meal | 5.0 |
| Poultry by-product meal | 6.0 |
| Whey | 6.0 |
| Vitamin premix | 2.0 |
| Choline chloride | 0.2 |
| DL-methionine | 0.2 |
| Mineral premix | 2.0 |
| Herring oil | 10.0 |
| Antibiotic premix* | 13.4 |
| | 100.0 |

* The several premixes employed were prepared as follows:

Control. The Antibiotic premix consisted solely of wheat middlings. The resulting diet was fed during the acclimitization to all treatment groups as well as to the control fish during the trial.

Tilmicosin Base. The Antibiotic premix was prepared as follows: 6.845 grams of tilmicosin base (5.97 grams of active tilmicosin) was mixed with 993.155 grams of wheat middlings. The resulting diet contained 800 mg of tilmicosin activity as tilmicosin base per kilogram of diet.

Tilmicosin Phosphate. The Antibiotic premix was prepared as follows: 20 grams of a mixture of 20% tilmicosin activity as tilmicosin phosphate on 80% ground corn cobs was further diluted with 650 grams of wheat middlings. The resulting diet contained 800 mg of tilmicosin activity as tilmicosin phosphate per kilogram of diet.

Table 7. Feed Intake, Weight Gain, and Feed Conversion, Comparing Tilmicosin Base and Phosphate Salt*

| Tilmicosin | Total feed intake (g) | Mean feeding rate (%BW) | Total tank weight gain (g) | Total tank weight gain (%BW) | Feed conversion |
|---|---|---|---|---|---|
| - (control) | 664.6 (37.5)[a] | 1.45 (0.08)[a] | 827.0 (40.4)[a] | 54.4 (2.4)[a] | 0.80 (0.01)[a] |
| as free base, 800 mg/kg | 658.9 (22.9)[a] | 1.40 (0.06)[a] | 831.0 (51.8)[a] | 53.5 (0.9)[a] | 0.79 (0.02)[a] |
| as phosphate, in concentration = 800 mg/kg free base | 625.5 (15.5)[a] | 1.38 (0.02)[a] | 802.3 (52.9)[a] | 53.7 (3.9)[a] | 0.78 (0.04)[a] |

* Mean values with the same alphabetic superscript were not statistically different at the 0.05 level.

Analysis of variance of the data did not show any significant differences among the three treatments. Thus, diets containing (1) tilmicosin (free base) at 800 mg/kg and (2) tilmicosin phosphate in a concentration equivalent to 800 mg/kg tilmicosin free base are palatable to Atlantic salmon, and are consumed at the same rate

as unmedicated diets. One fish from the tank receiving tilmicosin phosphate died; the cause of death could not be determined. One fish receiving nonmedicated feed exhibited a slight loss of equilibrium, but no effect on feeding behavior. Post-mortem examinations did not reveal any abnormalities in the fish receiving either form of tilmicosin.

## Claims

1. The use of a compound of Formula I

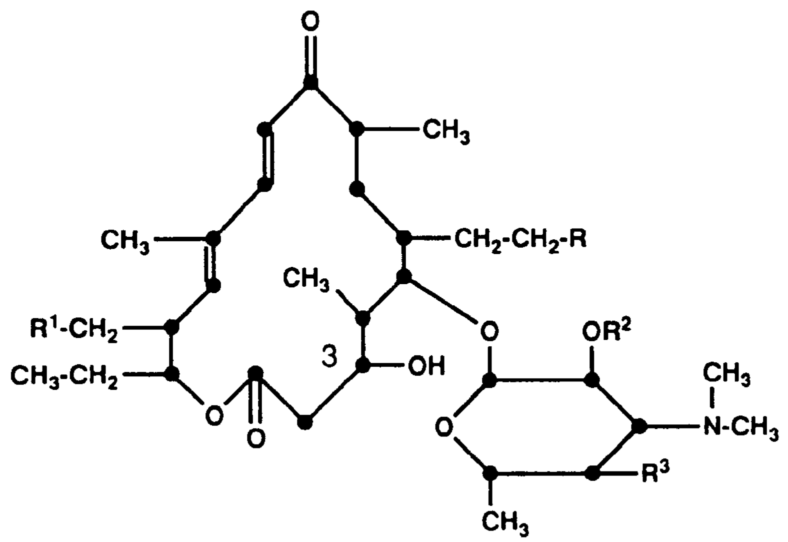

I

wherein

R is a saturated or unsaturated secondary amino group of the formula

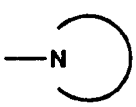

in which the nitrogen atom is part of an otherwise carbocyclic ring system selected from a monocyclic ring containing from 5 to 16 ring atoms or a bicyclic or tricyclic ring system containing from 8 to 20 ring atoms or such a group wherein one or more of the carbon atoms is substituted by $C_1$-$C_4$ alkyl, $C_2$-$C_4$ alkenyl, $C_2$-$C_4$ alkynyl, $C_1$-$C_4$ alkoxy, $C_1$-$C_4$ alkoxycarbonyl, hydroxyl, $C_1$-$C_4$ alkanoyloxy, halo, halo-$C_1$-$C_4$ alkyl, -N($C_1$-$C_4$ alkyl)$_2$, -N($CH_2$)$_m$,

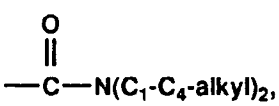, 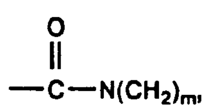,

cyano, ethylenedioxy, benzyl, phenyl, or phenyl substituted by from 1 to 3 substituents selected from nitro, halo, $C_1$-$C_4$-alkyl, $C_1$-$C_4$ alkoxy, hydroxy, amino, or mono- or di-($C_1$-$C_4$ alkyl)amino;

m is an integer from 4 through 7;

$R^1$ is

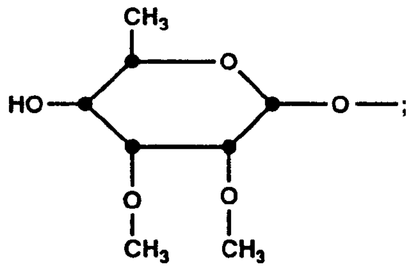

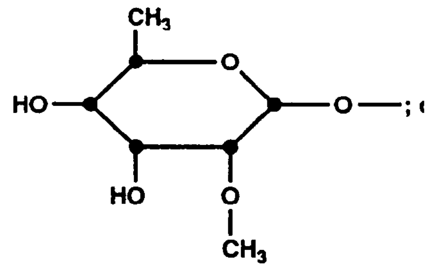

or

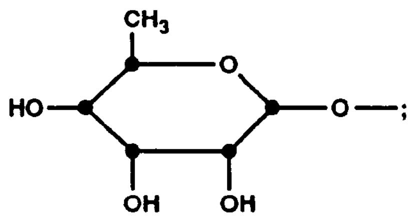

$R^2$ is hydrogen; $C_1$-$C_5$-alkanoyl or $C_1$-$C_5$-alkanoyl having from one to three halo substituents; benzoyl, phenylacetyl or phenylpropionyl or benzoyl, phenylacetyl or phenylpropionyl having from one to five halo or methyl or from one to two methoxyl, nitro or hydroxyl substituents;

$R^3$ is hydroxy; $C_1$-$C_5$ alkanoyloxy; $C_1$-$C_5$-alkanoyloxy having from one to three halo substituents; benzoyloxy, phenylacetoxy or phenoxyacetoxy or benzoyloxy, phenylacetoxy or phenoxyacetoxy having from one to five halo or methyl or from one to two methoxyl, nitro or hydroxyl substituents; or

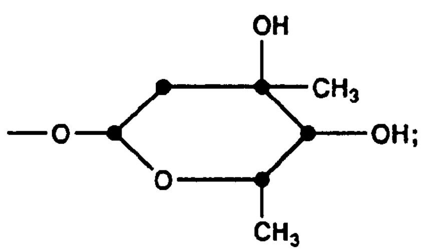

or a physiologically acceptable acid addition salt thereof, in the preparation of a medicament useful for treating or preventing infection by a gram-positive bacterium in an aquatic species which is a salmonid fish, a perciform fish, or a crustacean.

2. The use as claimed in Claim **1** wherein the compound of Formula I is tilmicosin or a physiologically acceptable salt thereof.

3. The use as claimed in Claim **1** or **2** for treating or preventing infection by *Renibacterium salmoninarum* in a salmonid fish.

4. The use as claimed in Claim **1** or **2** for treating or preventing infection by a *Streptococcus* spp. in a perciform fish.

European Patent Office

# EUROPEAN SEARCH REPORT

Application Number
EP 93 31 0258

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| Y,D | US-A-4 820 695 (DEBONO ET AL.)<br>* col.10 compounds no. D33,34,35 *<br>* column 14, line 1 - line 8 *<br>* column 19 *<br>* claims 1,52 *<br>--- | 1-4 | A61K31/445<br>A61K31/395<br>A61K31/365 |
| Y | CHEMICAL ABSTRACTS, vol. 104, no. 21, 26 May 1986, Columbus, Ohio, US;<br>abstract no. 183191w, 'The in vitro effect of macrolide antibiotics and lyncomycin on Streptococcus sp., the etiological agent odf streptococcal infection in yellowtail, Seriola quinqueradicata'<br>page 350 ;<br>* abstract *<br>& GYOBYO KENKYU<br>vol. 20, no. 4 , 1985<br>pages 453 - 7<br>--- | 1-4 | |
| Y | CHEMICAL ABSTRACTS, vol. 88, no. 5, 30 January 1978, Columbus, Ohio, US;<br>abstract no. 32165r, 'Antibiotic activity in fish'<br>page 45 ;<br>* abstract *<br>& JP-A-77 117 438 (YAMANOUCHI PHARM.) 1977<br>--- | 1-4 | TECHNICAL FIELDS SEARCHED (Int.Cl.5)<br>A61K |
| Y | VET.HUM.TOXICOL.<br>vol. 28, no. SP.1 , 1986<br>pages 11 - 17<br>'Antimicrobial and fish: a review of drugs used to treat bacterial diseases of channel catfish and rainbow trout'<br>* page 13, left column, line 26 - line 64 *<br>----- | 1-4 | |

The present search report has been drawn up for all claims

| Place of search | Date of completion of the search | Examiner |
|---|---|---|
| THE HAGUE | 15 April 1994 | Gerli, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)